# EUROPEAN PATENT APPLICATION

(11) **EP 4 537 819 A1**
(43) Date of publication of application: **16.04.2025**
(21) Application number: 23203563.4
(22) Date of filing: 13.10.2023
(51) Int. Cl.: A61K 31/167, A61K 31/196, A61P 29/00, A61K 9/00

(54) **ANALGESIC COMBINATION TREATMENT COMPRISING ORAL PARACETAMOL AND TOPICAL DICLOFENAC**

(71) Applicant: Haleon CH SARL, 1197 Prangins (CH)
(72) Inventor: Sood Sethi, Vidhu, 139234 Singapore (SG)
(74) Representative: Haleon Patent Department

(57) **Abstract**

The present invention relates to the use of compositions, methods and kits for the treatment of pain. In particular, the compositions, methods and kits are particularly useful, but not limited to, the treatment of pain associated with osteoarthritis (OA). Specifically, the pharmaceutical composition is a combination of two analgesics for treating pain comprising: a first pharmaceutical composition comprising paracetamol, and a second pharmaceutical composition comprising diclofenac, wherein the first pharmaceutical composition is a tablet or a caplet, and the second pharmaceutical composition is a topical formulation. When the two analgesics are concomitantly used at specific dosages, the combination produces analgesic pharmacological effects which are greater than when the two analgesics are used alone due to the complementary mechanisms of action of the two analgesics, targeting pain (paracetamol) and inflammation (topical diclofenac).

## Description

### Field of the Invention

The present invention relates to compositions, methods and kits for the treatment of pain. In particular, the compositions, methods and kits are particularly useful, but not limited to, the treatment of pain associated with osteoarthritis.

### Background of the Invention

Osteoarthritis (OA) is the most common degenerative disease of the joints and currently affects more than 527 million people globally, particularly the elderly population. Chronic pain is the hallmark symptom of OA that results in significant disability and reduced quality of life in affected individuals and is also the major reason for them to seek medical care. However, despite the huge burden associated with OA, pain management continues to remain sub-optimal since current symptomatic therapies often exhibit modest efficacy (e.g., paracetamol), unfavorable safety profile (e.g., oral non-steroidal anti-inflammatory drugs (NSAIDs)) and in some cases increased risk of addiction and overdose (e.g. opioids).

Growing evidence indicates that OA pain is a multi-mechanistic phenomenon which encompasses inflammatory and non-inflammatory pain pathways at peripheral and central levels of the nervous system. Whilst the identification of disease-modifying treatments is an ongoing endeavour in chronic conditions such as OA, a promising approach is the use of rational drug combinations, which act through different mechanisms to provide more effective pain relief with a more favourable risk-benefit ratio.

Paracetamol is one of the most commonly used analgesic and antipyretic medications across the world and is also included on the World Health Organization's list of essential medicines as an effective and safe medicine needed in a health system. Historically, it has been used as a first-line pain medication for OA, but recent reports from a wider range of clinical trials suggest that its use as a single agent results in modest efficacy. This finding may reflect its mechanism of action, which is mainly centrally mediated via the descending serotonergic pathways with minimal influence on peripheral pathways. On the other hand, topical NSAIDs, including diclofenac, have emerged as useful treatment options for OA individuals with contraindications to oral NSAIDs. They act primarily by targeting peripheral mechanisms of pain and inflammation by mediating cyclooxygenase inhibition in the skin and soft tissue, including cartilage^{12,13,14}.

Effective management of mild-to-moderate OA pain continues to remain suboptimal despite the availability of the pharmacologic treatments discussed above.

Thus, there remains a need for further innovation in this area to offer science-based treatment options to individuals suffering from osteoarthritis pain which provide adequate reliefand are well-tolerated.

### Summary of the Invention

In a first aspect of the invention, there is provided a pharmaceutical combination for treating pain comprising: a first pharmaceutical composition comprising paracetamol, and a second pharmaceutical composition comprising diclofenac, wherein the first pharmaceutical composition is a tablet or a caplet, and the second pharmaceutical composition is a topical formulation.

Particularly, the first pharmaceutical composition is a tablet comprising 500mg of paracetamol, and one or more pharmaceutically acceptable diluents, excipients or carriers.

Alternatively, the first pharmaceutical composition is a tablet comprising 1000mg of paracetamol, and one or more pharmaceutically acceptable diluents, excipients or carriers.

Particularly, the second pharmaceutical composition is an emulgel comprising 1.16% of diclofenac diethylamine salt (corresponding to 1% diclofenac sodium), and one or more pharmaceutically acceptable diluents, excipients or carriers. Such a formulation is commercially available as Voltaren^{®} Emulgel^{®}.

Alternatively, the second pharmaceutical composition is an emulgel comprising 2.32% of diclofenac diethylamine salt (corresponding to 2% diclofenac sodium), and one or more pharmaceutically acceptable diluents, excipients or carriers. Such a formulation is described for example in EP2214642B1, incorporated by reference herein and is commercially available as Voltaren^{®} Forte or Voltaren^{®} Osteo Gel 12 Hourly.

In a second aspect of the invention, there is provided a method for treating pain, namely pain associated with osteoarthritis, in a human subject, wherein the method comprises administering a first pharmaceutical composition comprising paracetamol, and a second pharmaceutical composition comprising diclofenac, and wherein the first pharmaceutical composition is a tablet or a caplet, and the second pharmaceutical composition is a topical formulation.

In a third aspect of the invention, there is provided a kit for treating pain comprising at least two pharmaceutical compositions, wherein the kit is adapted to allow access to each composition at different times, wherein a first composition is a tablet or caplet comprising paracetamol and one or more pharmaceutically acceptable diluent, excipient or carrier; and wherein a second composition is a topical formulation comprising diclofenac and one or more pharmaceutically acceptable diluent, excipient or carrier. Preferably, the topical formulation is an emulgel or a clear gel.

Optionally the kit also includes written instructions directing the user to administer one or more tablets or caplets at a particular time of the day or event, and to apply a certain amount of the topical formulation at a particular time of the day or event. Preferably, the written instructions direct the user to administer the first composition three or four times per day, with a total daily dose not exceeding 4000mg of paracetamol for an adult, and to administer the topical formulation 2 to 4 times per day, depending on the diclofenac concentration, with a total daily dose not exceeding 16 g/day for diclofenac diethylamine emulgel 1.16% and 4 g/day for diclofenac diethylamine emulgel 2.32%, respectively.

Preferably, the kit comprises a blister card containing the first composition, and a tube containing the second composition. In some embodiments, one or two of the tablets or caplets are administered four times per day at a particular time of day or event, and the topical composition is administered at least twice at the same time as the administration of the tablets or caplets. Preferably, the topical formulation is administered at every second administration of the first composition.

### Brief description of Figures

Figure 1 shows the observed ratio of the beneficial effect of systemic combination comprising paracetamol and topical diclofenac on pain score reduction at 6 to 72 hours, compared to either drug alone across 11 acute pain studies by use of opioid patient-controlled analgesia (PCA) vs. no PCA.
Figure 2 shows simulated placebo-adjusted opioid PCA use (in mg) for paracetamol and diclofenac monotherapies and their combination assuming a typical placebo response (64.7 mg).

### Description of the Invention

The term "comprising" encompasses "including" e.g. a composition "comprising" X may include something additional e.g. X + Y. In some implementations, the term "comprising" refers to the inclusion of the indicated active agent, such as paracetamol, as well as inclusion of other active agents, and pharmaceutically acceptable carriers, excipients, emollients, stabilizers, etc., as are known in the pharmaceutical industry.

All references or patent applications cited within this patent specification are incorporated by reference herein. In order that this invention may be better understood, the following examples are set forth. These examples are for purposes of illustration only and are not to be construed as limiting the scope of the invention in any manner.

Paracetamol and topical diclofenac exhibit complementary mechanisms of action, targeting pain and inflammation, respectively. Therefore, use of this analgesic combination could be a promising tool in achieving effective relief of mild to moderate OA pain, particularly in patients who are contraindicated to oral NSAIDs or are intolerant of oral opioids. The analgesic combination could also help patients achieve adequate pain relief and potentially limit repeated supratherapeutic ingestions of paracetamol which occur in case of insufficient pain relief and are associated with worse clinical outcomes than isolated paracetamol overdose.

The efficacy of the analgesic combination comprising of oral paracetamol and topical diclofenac gel was investigated and compared to paracetamol and diclofenac monotherapies in three unpublished clinical studies.

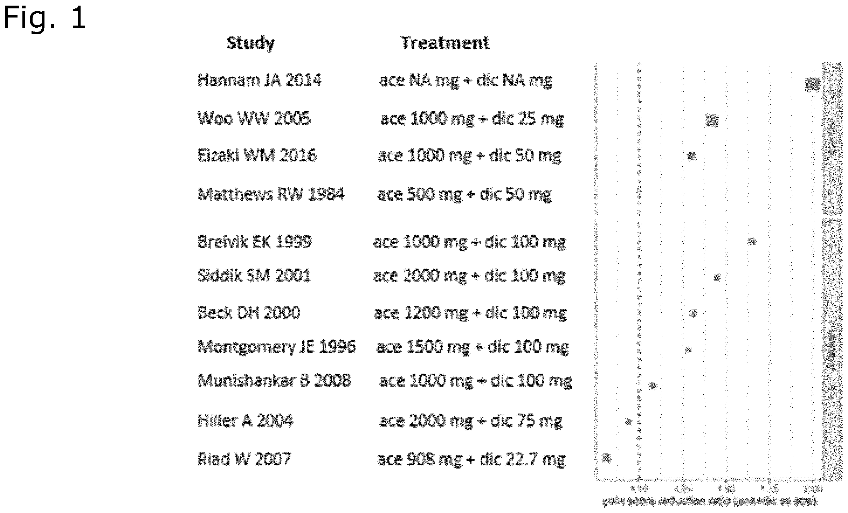

The first study used pharmacodynamic modelling and simulation (hereafter called M&S study) to assess the efficacy of the analgesic combination against either monotherapy. In the M&S study, exploratory analysis of data showed the analgesic combination to exhibit beneficial pain score reduction in 8 out of the 11 included randomized clinical studies (RCTs) versus paracetamol monotherapy and in 5 out of the 11 RCTs versus diclofenac monotherapy. These results are shown in Fig. 1 which shows the observed ratio of the beneficial effect of systemic combination comprising paracetamol and topical diclofenac on pain score reduction at 6 to 72 hours, compared to either drug alone across 11 acute pain studies by use of opioid patient-controlled analgesia (PCA) vs. no PCA. In Fig. 1, the vertical dash line (ratio= 1) indicates no beneficial effect of the combination compared to either drug alone in the same study. The size of square represents the sample size in the study arm. ace: acetaminophen; dic: diclofenac; OPIOID P: Opioid PCA; NA: Not applicable

Additionally, the M&S study demonstrated opioid sparing benefit with the combination compared to either drug. Specifically, the model showed about 32% less opioids use with the combination than paracetamol monotherapy based on point of estimation. However, the beneficial effect of the combination effect was less pronounced vs. diclofenac monotherapy. The typical treatment effect on mean opioid PCA consumption difference from placebo, and percent difference from paracetamol is shown in Table 1.

**Table 1 Typical treatment effect on mean opioid PCA consumption difference from placebo, and percent difference from paracetamol.**

| **Treatment** | **Model predicted mean placebo-adjusted opioid PCA use (in mg) with 95% CI** | **Percent difference from paracetamol (%)** |
|---|---|---|
| **Systemic paracetamol** | -18.93 (-29.47, -8.33) | - |
| **Systemic diclofenac** | -28.41 (-39.15, -17.68) | -20.7 (-32.86, -8.45) |
| **Systemic paracetamol** + **diclofenac** | -33.87 (-44.43, -22.26) | -32.62 (-47.59, -19.1) |

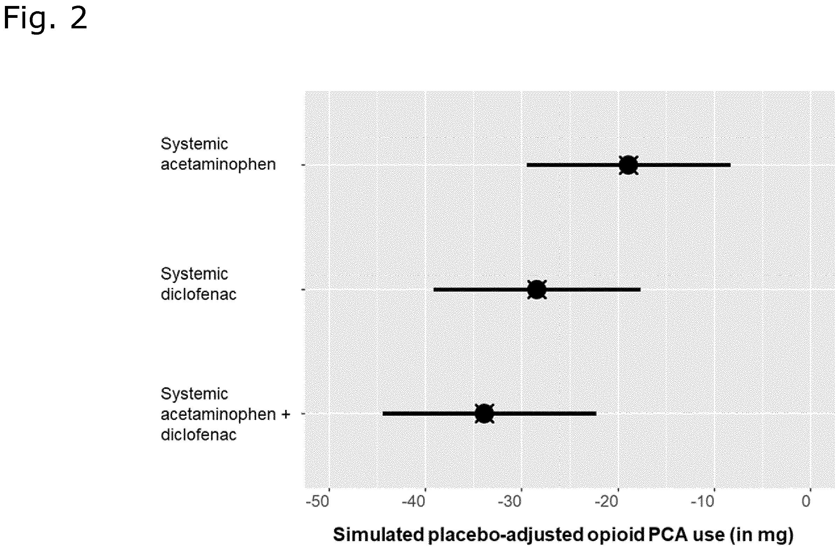

These results are also shown in Fig. 2, which shows simulated placebo-adjusted opioid patient-controlled analgesia (PCA) use (in mg) for paracetamol and diclofenac monotherapies and their combination assuming a typical placebo response (64.7 mg). In Fig. 2, symbols indicate maximum likelihood model predictions and error bars represent 95% confidence interval (CI) of resampling parameter estimates from the final model variance-covariance matrix 1,000 times.

The second study is a retrospective analysis of UK Biobank using real world big data and machine learning technologies to investigate the benefits of the analgesic combination versus monotherapies on OA-related outcomes, including time from diagnosis to total joint replacement (TJR) surgery, use of opioids-based analgesics and depression (hereafter called UK Biobank study). The UK Biobank is a large-scale biomedical database and research resource containing in-depth genetic and health information from around 500,000 UK participants from 2006 to 2010. Preliminary results of the UK Biobank study have also confirmed the beneficial effect of the analgesic combination observed in the M&S study. The UK Biobank study showed the analgesic combination to be significantly associated with increased time from OA diagnosis to joint replacement surgery and ~66% reduction in surgeries, when compared with paracetamol alone, in knee OA patients [effect estimate (standard error) for the analgesic combination -1.0837 (0.5089), p < 0.05 vs. 0.1762 (0.1166), non-significant for paracetamol alone]. Additionally, the analgesic combination was significantly associated with longer time from OA diagnosis to opioids use and ~45% reduction in transition to opioids use [effect estimate (standard error) for the analgesic combination -0.5970 (0.2956), p < 0.05 vs. 18.8211 (455.4795), non-significant for paracetamol alone]. Moreover, the analgesic combination was significantly associated with lower patient health questionnaire-9 (PHQ-9) depression scores (~4-8 points reduction in PHQ-9 depression score) when compared with paracetamol monotherapy [effect estimate (standard error) for the analgesic combination -5.2760 (2.0745), p=0.0112 vs. 1.4925 (0.7503), p=0.0472 for paracetamol alone].

Regarding the safety profile of the combination, while topical diclofenac is considered as well-tolerated in OA pain management due to its low systemic exposure, concerns of liver toxicity with paracetamol remain. Therefore, a separate modelling and simulation study was conducted to assess the risk of liver toxicity with paracetamol use by measuring deviation from upper limit of normal (ULN) of alanine aminotransferase or aspartate aminotransferase, namely >0-1 ULN, >1.5-2 ULN, and >3 ULN representing mild, moderate, and severe risk of liver abnormality (hereafter called M&S-2 study). The M&S-2 study showed short-term (~8-16 weeks) administration of paracetamol (1500-4000 mg/day) to exhibit 23%, 1.35% and 0.01% increased risk for mild, moderate and severe liver injury, respectively, versus background or placebo rate. Although a 23% increased risk appears numerically large, mild elevation in transaminases is not considered clinically meaningful because of self-healing capacity of the liver. Therefore, it can be concluded that paracetamol use for short-term (< 16 weeks) at standardized dosages (≤4000gm/day) is associated with very low risk of clinically meaningful liver injury. The findings of the M&S-2 study are in agreement with most OA clinical practice guidelines that recommend short-term or episodic use of paracetamol at < 3 g/day and/or not exceeding 4 g/day, e.g., in elderly subjects, with joint consideration of its analgesic effect and potential tolerability issues

Given the good tolerability profile of diclofenac gel, the findings thus support the combination of paracetamol and topical diclofenac gel to possess high tolerability, at least over short-term, when used in the treatment of mild-to-moderate OA pain.

### Examples:

### Example 1:

One or two standard 500mg paracetamol tablet are administered 4 times/day, i.e. up to a daily dose of 4000mg. A diclofenac diethylamine 1.16% emulgel is applied on joints suffering from osteoarthritis with each paracetamol administration, with 2-4g of the product being applied each time, depending on the size of the joint.

The two products can be packed together in a combination pack containing one or two blister cards containing a total of 16 500mg paracetamol tablets and a 30g or 50g tube of the diclofenac diethylamine 1.16% emulgel .

Alternatively, the two products can be packed separately, but sold as a kit or a bundle with the recommendation to use them for a combination treatment as indicated above.

### Example 2:

One or two standard 500mg paracetamol tablet are administered 4 times/day, i.e. up to a daily dose of 4000mg. A diclofenac diethylamine 2.32% emulgel is applied on joints suffering from osteoarthritis with every second paracetamol administration, i.e 2 times a day, with 2-4g of the product being applied each time, depending on the size of the joint.

The two products can be packed together in a combination pack containing one or two blister cards containing a total of sixteen 500mg paracetamol tablets and a 20g, 30g or 50g tube of the diclofenac diethylamine 2.32% emulgel.

Alternatively, the two products can be packed separately, but sold as a kit or a bundle with the recommendation to use them for a combination treatment as indicated above.

## Claims

1. Pharmaceutical combination product for treating pain comprising: a first pharmaceutical composition comprising paracetamol, and a second pharmaceutical composition comprising diclofenac, wherein the first pharmaceutical composition is for oral use, and the second pharmaceutical composition is a topical formulation.

2. The combination product of claim 1, wherein the first pharmaceutical composition is a tablet comprising 500mg of paracetamol, and one or more pharmaceutically acceptable diluents, excipients or carriers.

3. The combination product of claim 1 or 2, wherein the first pharmaceutical composition is a tablet comprising 1000mg of paracetamol, and one or more pharmaceutically acceptable diluents, excipients or carriers.

4. The combination product of one of the preceding claims, wherein the second pharmaceutical composition is an emulgel comprising 1.16% of diclofenac diethylamine salt (corresponding to 1% diclofenac sodium), and one or more pharmaceutically acceptable diluents, excipients or carriers.

5. The combination product of one of the preceding claims, wherein the second pharmaceutical composition is an emulgel comprising 2.32% of diclofenac diethylamine salt (corresponding to 2% diclofenac sodium), and one or more pharmaceutically acceptable diluents, excipients or carriers.

6. A method for treating pain, namely pain associated with osteoarthritis, in a human subject, wherein the method comprises administering a first pharmaceutical composition comprising paracetamol, and a second pharmaceutical composition comprising diclofenac, and wherein the first pharmaceutical composition is administered orally, and the second pharmaceutical composition is administered topically.

7. The method of claim 6, wherein the first pharmaceutical composition is a tablet comprising 500mg of paracetamol, and the second pharmaceutical composition is a gel or an emulgel.

8. A kit for treating pain comprising at least two pharmaceutical compositions, wherein a first composition is a tablet or caplet comprising paracetamol and one or more pharmaceutically acceptable diluent, excipient or carrier; and wherein a second composition is a topical formulation comprising diclofenac and a pharmaceutically acceptable diluent, excipient or carrier.

9. The kit according to claim 8, wherein the kit also includes written instructions directing the user to administer one or more tablets or caplets three to four times a day at a particular time of the day or event, and to apply a certain amount of the topical formulation at the same time of the day or event, with the topical formulation containing 1.16% of diclofenac diethylamine salt or 1% of diclofenac sodium salt.

10. The kit according to claim 8, wherein the kit also includes written instructions directing the user to administer one or more tablets or caplets four times a day at a particular time of the day or event, and to apply a certain amount of the topical formulation every second and fourth time when the tablets or caplets are administered, with the topical formulation containing 2.32% of diclofenac diethylamine salt or 2% of diclofenac sodium salt.
